# EUROPEAN PATENT APPLICATION

(11) **EP 4 681 700 A1**
(43) Date of publication of application: **21.01.2026**
(21) Application number: 23928479.7
(22) Date of filing: 17.03.2023
(51) Int. Cl.: A61K 8/64, A61K 38/00, C12N 9/12, C07K 7/06

(54) **GSE24.2-DERIVED PEPTIDE AND USES THEREOF**

(71) Applicant: Consejo Superior De Investigaciones Científicas - CSIC, 28006 Madrid (ES); Universidad Autónoma de Madrid, 28049 Madrid (ES); Consorcio Centro de Investigación Biomédica en Red, 28029 Madrid (ES)
(72) Inventor: GUILLEN MORALES, Paula, 28029 Madrid (ES); PERONA ABELLON, Rosario, 28029 Madrid (ES); SASTRE GARZON, Leandro, 28029 Madrid (ES); FERNANDEZ-VARAS, Beatriz, 28029 Madrid (ES); GUTIERREZ RODRIGUEZ, Marta, 28006 Madrid (ES); MARTIN MARTINEZ, Mercedes, 28006 Madrid (ES); GUERRERO LOPEZ, Rosa, 28029 Madrid (ES)
(74) Representative: Pons IP
(86) International application number: PCT/ES2023/070172
(87) International publication number: WO 2024/194500

(57) **Abstract**

The present invention relates to a GSE24.2 derived peptide and its related uses. In particular, the present invention provides a peptide comprising the amino acid sequence with SEQ ID NO. 1 with the condition that said peptide does not have the amino acid sequence SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11 or SEQ ID NO. 64, and uses thereof in the prevention and/or treatment of individuals with diseases caused by cellular DNA damage or telomer shortening, for example.

## Description

The present invention is within the field of biomedicine. Specifically, it relates to a GSE24.2 derived peptide and uses related to cellular ageing, as well as in the prevention and/or treatment of diseases or disorders caused by cellular DNA damage, telomere shortening, or inflammation.

### STATE OF THE ART

GSE24.2 is a peptide corresponding to the dyskerin protein, which is part of the telomerase complex. GSE24.2 is capable of reactivating telomerase in pathological and physiological situations; in telomerase-defective diseases GSE24.2 activity increases the viability of patient-derived cells.

Patent application WO 2007/090911 A1 entitled "Nucleotide sequence and peptides GSE24.2 of dyskerin, which can induce telomerase activity, method for obtaining same, therapeutic compositions and applications thereof" discloses the use of GSE24.2 to reactivate telomerase activity or in a medicament or pharmaceutical composition. In said international patent application, a sequence of 55 amino acids is identified as the original functional unit.

On the other hand, patent application WO 2015/059338 A1 entitled "Peptides derived from GSE24.2 for treating diseases caused by oxidative stress and damage to DNA" describes GSE24.2 derived peptides capable of re-establishing the normal redox equilibrium of a cell and/or of correcting DNA damage.

An important parameter to be taken into account in the development of molecules with therapeutic potential is the size of the molecules because, generally, a larger size is linked to worse membrane permeability, metabolic instability, higher costs, or worse bioavailability. Therefore, in view of the foregoing, there is a need in the state of the art to provide peptides having a smaller size than GSE24.2, alternatives to the pre-existing ones.

### DESCRIPTION OF THE INVENTION

It is known in the state of the art that the GSE24.2 peptide and fragments derived therefrom, such as the GSE4 peptide, described previously in patent application WO 2015/059338 A1, reduces or decreases cellular DNA structure damage.

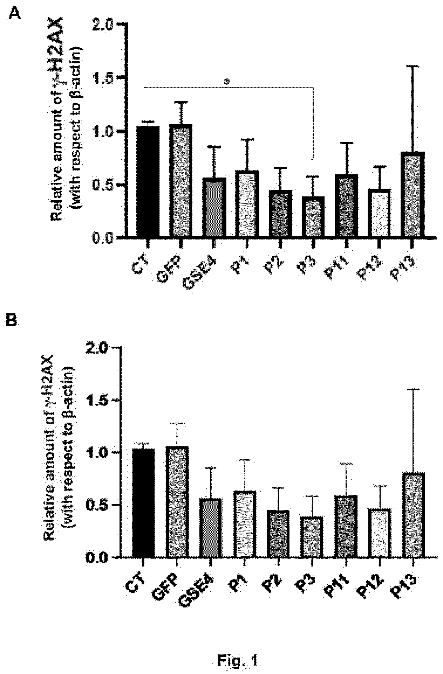

In the present invention, the inventors have identified a peptide with an even shorter amino acid sequence, SEQ ID NO. 1, capable of causing a decrease in DNA damage that is even greater than that caused, for example, by the GSE4 peptide. In particular, the inventors have demonstrated that said peptide causes a decrease in the levels yH2AX, which are indicators of the existence of DNA damage, both in culture control conditions and after cell treatment with bleomycin, with said decrease being greater than that caused by the expression of the GSE4 peptide described previously (Example 1). On the other hand, the inventors have also demonstrated that said decrease is also greater than that caused by other dyskerin protein-derived or related peptides, such as the GSE4 peptide (Example 1; Figures 1A and 1B). Other related peptides do not show this degree of decrease in damage, specifically, other assayed peptides that are shorter than P3 do not cause a decrease in the expression of γH2AX, so it is not obvious that peptides shorter than GSE4 maintain the same biological activity (Example 1). Furthermore, other effects of the peptide with SEQ ID NO. 1, are an increase in the expression of TERT and TERC genes, encoding components of the telomerase complex, indicating the peptide's activity in cellular processes in which telomere shortening (Example 2) is present, as well as effects on transforming growth factor-beta (TGF-β) response inhibitors, as shown in Example 3, being therapeutically applicable in fibrosis and ageing processes in which said molecule is involved, and proinflammatory response modulation (Example 4).

The development of peptides with shorter amino acid sequences in the present invention with activities such as cellular DNA damage reduction has advantages associated with higher solubilisation, simpler folding, and improved cellular uptake, favouring their administration in the treatment and/or prevention of related diseases. Furthermore, having shorter peptides compared to the GSE24-2 peptide and other derived fragments of greater length prevents the appearance of unwanted effects due to the interaction of the rest of the molecule with other cellular effectors.

On this basis, the inventors have developed a number of inventive aspects that will be described below:

### Peptide of the invention

One aspect of the invention relates to a peptide comprising or consisting of the amino acid sequence SEQ ID NO. 1, hereinafter the "peptide of the invention", with the proviso that said peptide does not have the amino acid sequence SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11 or SEQ ID NO. 64.

A list of the sequences and the correspondences with the peptides described in this invention is shown below in the Table 1:

**Table 1. Correspondence between the peptides and the described sequences.**

| Peptide name | Sequence | No. of amino acids | SEQ ID NO. |
|---|---|---|---|
| P3 | DKPSNPSSHE | 10 | 1 |
| GSE4 | GFINLDKPSNP | 11 | 2 |
| GSE24.2 | GFINLDKPSNPSSHEVVAWIRRILRVEKT GHSGTLDPKVTGCLIVCIERATRLVK | 55 | 3 |
| GSE1 | GFINLDKPSNPSSHEVV | 17 | 4 |
| GSE2 | GFINLDKPSNPSSHEVVA | 18 | 5 |
| GSE3 | GFINLDKPSNPSSHEVVAW | 19 | 6 |
| GSE5 | GFINLDKPSNPSSHEVVAWIRRILR | 25 | 7 |
| GSE24.2NLS1-3 | GFINLDKPSNPSSHEVVAWIRRILRVEKT GHSGTLDPKVTGCLIVCIERATRLVKKR KR | 59 | 8 |
| GSE24.2NLS2-3 | GFINLDKPSNPSSHEVVAWIRRILRVEKT GHSGTLDPKVTGCLIVCIERATRLVKKK EKKKSK | 63 | 9 |
| GSE24.2NLS1-5 | KRKRGFINLDKPSNPSSHEVVAWIRRIL RVEKTGHSGTLDPKVTGCLIVCIERATR LVK | 59 | 10 |
| GSE24.2NLS2-5 | KKEKKKSKGFINLDKPSNPSSHEVVAWI RRILRVEKTGHSGTLDPKVTGCLIVCIER ATRLVK | 63 | 11 |
| P1 | NLDKPSNP | 8 | 12 |
| P2 | NLDKPSNPSSHE | 12 | 13 |
| P11 | GFINLDK | 7 | 14 |
| P12 | DKPSNP | 6 | 15 |
| P13 | EHSSPNSPKD | 10 | 16 |
| P3.NLS1 | DKPSNPSSHEKRKR | 14 | 17 |
| P3.NLS2 | DKPSNPSSHEKKEKKKSK | 18 | 18 |
| Dyskerin | MADAEVIILPKKHKKKKERKSLPEEDVAEIQHAEE FLIKPESKVAKLDTSQWPLLLKNFDKLNVRTTHYT PLACGSNPLKREIGDYIRTGFINLDKPSNPSSHEV VAWIRRILRVEKTGHSGTLDPKVTGCLIVCIERAT RLVKSQQSAGKEYVGIVRLHNAIEGGTQLSRALE TLTGALFQRPPLIAAVKRQLRVRTIYESKMIEYDP ERRLGIFWVSCEAGTYIRTLCVHLGLLLGVGGQM QELRRVRSGVMSEKDHMVTMHDVLDAQWLYDN HKDESYLRRVVYPLEKLLTSHKRLVMKDSAVNAI CYGAKIMLPGVLRYEDGIEVNQEIVVITTKGEAIC MAIALMTTAVISTCDHGIVAKIKRVIMERDTYPRK WGLGPKASQKKLMIKQGLLDKHGKPTDSTPATW KQEYVDYSESAKKEVVAEVVKAPQVVAEAAKTA KRKRESESESDETPPAAPQLIKKEKKKSKKDKKA KAGLESGAEPGDGDSDTTKKKKKKKKAKEVELV SE | 514 | 64 |

Therefore, the peptide of the invention, comprising, or consisting of, the amino acid sequence SEQ ID NO. 1, is a fragment of the sequence of the GSE24.2 peptide (SEQ ID NO. 3), being a peptide of a smaller size alternative to those described in the state of the art, which furthermore has, as mentioned above, different cellular ageing-related activities, such as reduction of/protection against telomere shortening or cellular DNA damage, showing in an improved effect with respect to GSE4 (SEQ ID NO. 2) described previously in patent application WO 2015/059338 A1 as the minimal unit responsible for GSE24.2 activity in decreasing cellular DNA structure damage. Furthermore, the peptide of the invention also shows an improved effect with respect to other alternative peptides derived from GSE24.2 and GSE4 peptides that have also been analysed.

As mentioned above, the peptide of the invention decreases DNA structure damage in a cell. Therefore, a particular embodiment provides the peptide of the invention, wherein said peptide is capable of decreasing DNA damage in a cell.

Cellular DNA damage can be evaluated by means of methods known in the state of the art such as, for example, by means of determining the presence of the phosphorylated histone H2AX variant, referred to as yH2AX, by Western blot. When DNA damage such as DNA strand breakage occurs, one of the quickest responses consists of the incorporation of yH2AX to the chromatin in the damaged DNA region, so analysing its levels allows cellular DNA damage to be evaluated.

Furthermore, as also been mentioned above, the peptide of the invention exhibits other activities, such as IL-6-mediated proinflammatory response reduction activity, telomere shortening reduction or protection activity, or TGF-β- and/or MCP1-mediated fibrosis reduction activity.

Therefore, a particular embodiment, alone or in combination with other particular embodiments, provides the peptide of the invention, wherein said peptide exhibits at least one activity selected from the list consisting of: proinflammatory response reduction activity, preferably IL-6-mediated, reducing or protecting against telomere shortening, TGF-β- and/or MCP1-mediated fibrosis reduction activity, and any combination thereof.

In the context of the present invention, peptide is understood to mean that molecule formed by the attachment of amino acids by means of peptide bonds. Nevertheless, in a particular embodiment, the peptide of the invention has a length of 10 to 150 amino acids, more particularly 10 to 100 amino acids (including the ends), more particularly 10 to 54 amino acids (including the ends), 10 to 40 amino acids (including the ends), 10 to 30 amino acids (including the ends), or 10 to 20 amino acids (including the ends). In an even more particular embodiment, the peptide of the invention has a length of 10 to 14 amino acids (including the ends), even more particularly 10, 11, 12, 13,. or 14 amino acids. As understood by a person skilled in the art, generally, the smaller the peptide, the easier it will be to administer and the fewer side effects it will have.

The peptide of the invention can be obtained by techniques widely known in the state of the art. Examples of techniques for obtaining peptides include, but are not limited to, chemical or biological synthesis, genetic recombination, or expression of polynucleotides encoding the peptide of the invention.

Additionally, the carboxyl and amino-terminal ends of the peptide of the invention may be protected against proteolysis. For example, the amino-terminal end may be in the form of an acetyl group and/or the carboxyl-terminal end may be in the form of an amide group. It is also possible to carry out internal modifications of the peptides so that they are resistant to proteolysis, for example, in which at least one peptide bridge -CONH- is modified and replaced by a reduced bond (CH2NH), a retroinverso bond (NHCO), an oxymethylene bond (CH2-O), a thiomethylene bond (CH2-S), a ketomethylene bond (CO-CH2), a hydroxyethylene bond (CHOH-CH2), a bond (N-N), an E-alkene bond, or a -CH=CH- bond. The amino acids of the peptide of the invention may be in D-form, which can give rise to peptides resistant to proteolysis. Peptides can also be stabilised by intramolecular cross-linking, for example, by modifying at least two amino acid residues with olefinic side chains, preferably, C3-C8 alkenyl chains, preferably, pentel-2-yl chains, followed by cross-linking of the chains as described in the so-called "staple" technology (Walensky et al., 2004, Science 205: 1466-1470). All these peptides chemically modified to resist proteolysis are also contemplated in the present invention.

Additional modifications to the peptide of the invention comprise covalent bonding to a polyethylene glycol (PEG) molecule at its carboxyl-terminal end or to a lysine residue, in order to lessen urinary elimination thereof and the therapeutic dose and to increase the half-life of the peptide in blood plasma. The half-life of the peptide can also be increased by including the peptide in a biodegradable and biocompatible polymer material to form microspheres that are used as a drug delivery system. Polymers and copolymers are, for example, poly (D, L-lactide-co-glycolic acid) or PLGA. The techniques and procedures for manufacturing lipid microspheres or nanocapsules for use in drug administration are widely known to the person skilled in the art.

Furthermore, the use of biotin for distributing and/or targeting molecules to a tumour cell, in which genetic damage is a common molecular mark or characteristic, is widely known in the state of the art because biotin receptors are overexpressed in cells of this type. Therefore, the attachment of molecules such as, for example, peptides, to biotin, may improve their distribution or targeting to a tumour cell. Therefore, in a particular embodiment, alone or in combination with other particular embodiments, the peptide of the invention is covalently attached to biotin.

Likewise, the peptide of the invention may comprise in its amino acid sequence conservative amino acid substitutions which maintain the capacity to decrease DNA damage in a cell and/or other activities such as IL-6-mediated proinflammatory response reduction activity, reducing or protecting against telomere shortening, or TGF-β- and/or MCP1-mediated fibrosis reduction activity. Therefore, in the context of the present invention, peptides derived from the peptide of the invention, called "variants", are also contemplated, where although they do not exhibit 100% sequence identity with the peptide of the invention, maintain said capacity, since the amino acids have been replaced by others that are biologically similar. Assays to find out whether a peptide derived from the peptide of the invention has the capacity to decrease DNA structure damage in a cell, or other activities, such as IL-6-mediated proinflammatory response reduction activity, reducing or protecting against telomere shortening , or TGF-β- and/or MCP1-mediated fibrosis reduction activity, are described in the section of Examples.

Therefore, in a particular embodiment, the peptide of the invention comprises or consists of an amino acid sequence with a sequence identity of at least 70, 75, 80, 85, 90, 91, 92, 93, 94, 95, 96, 97, 98, or 99% with SEQ **ID** NO. 1.

In another particular embodiment, the peptide of the invention comprises, or consists of, the amino acid sequence SEQ ID NO. 13. This amino acid sequence SEQ **ID** NO. 13, has two additional amino acids with respect to SEQ **ID** NO. 1, that is, it in turn comprises SEQ ID NO. 1. As mentioned above, the peptide of the invention, as a condition, does not have the amino acid sequence SEQ ID NO. 3, SEQ **ID** NO. 4, SEQ **ID** NO. 5, SEQ ID NO. 6, SEQ ID NO. 7, SEQ ID NO. 8, SEQ ID NO. 9, SEQ ID NO. 10, SEQ ID NO. 11 or SEQ ID NO. 64.

Taking into account that the action of the peptide of the invention can be carried out in the cell nucleus, to facilitate its expression or localisation in the nucleus and/or to improve the activity thereof, said peptide may further comprise at least one nuclear localisation sequence (NLS) attached to at least one of the carboxyl- or amino-terminal ends of said fragment, and more preferably at the carboxyl-terminal end.

Therefore, in a particular embodiment, alone or in combination with other particular embodiments, the peptide of the invention comprises, at least, one nuclear localisation sequence attached to at least one of the carboxyl- or amino-terminal ends of its amino acid sequence.

In the field of the present invention, nuclear localisation sequence is understood to mean a group of amino acids capable of directing the localisation of the peptide to which they are attached in the cell nucleus; said sequence may be both a nuclear transport sequence and a nucleolar transport sequence. Preferably, the nuclear localisation sequence is a nuclear localisation sequence found in the DKC1 gene(dyskerin pseudouridine synthase 1; NG_009780.1_NCBI RefSeqGene) from which the GSE24.2 sequence is derived such as, for example, nuclear transport sequence KRKR (NLS1) or nucleolar transport sequence KKEKKKSK (NLS2; SEQ ID NO. 20), and more preferably, it is the nuclear transport sequence KRKR (NLS1; SEQ ID NO. 19).

Therefore, in a more particular embodiment, the nuclear localisation sequence comprises the sequence SEQ ID NO. 19 [KRKR] or sequence SEQ ID NO. 20 [KKEKKKSK].

In another particular embodiment, the peptide of the invention comprises or consists of the amino acid sequence SEQ ID NO. 17 or SEQ ID NO. 18.
SEQ ID NO. 17 - DKPSNPSSHEKRKR
SEQ ID NO. 18 - DKPSNPSSHEKKEKKKSK

### Polynucleotide of the invention

As indicated above, the peptide of the invention can be obtained by techniques widely known in the state of the art, such as the expression in a cell of the polynucleotide encoding the peptide of the invention, and the subsequent isolation thereof. Therefore, in another aspect, the invention relates to a polynucleotide, hereinafter the "polynucleotide of the invention", which encodes the peptide of the invention.

The term "polynucleotide", as used in the present invention, refers to a polymeric form of nucleotides of any length, formed by ribonucleotides and/or deoxyribonucleotides. The term includes both single-stranded and double-stranded polynucleotides, as well as modified polynucleotides (methylated, protected and similar). The polynucleotide of the invention may be a DNA, an RNA, or a cDNA.

Taking into account that the action of the polypeptide encoded by the polynucleotide of the invention can be carried out in the cell nucleus, in a manner similar to what has been indicated for the peptide of the invention, the sequence of the polynucleotide of the invention may further comprise at least one sequence encoding a nuclear localisation sequence (NLS) attached to at least one of the 3' or 5' ends of the sequence encoding the peptide of the invention, and more preferably attached to the 3' end.

In a particular embodiment, alone or in combination with other particular embodiments, the polynucleotide of the invention has a length of 36 to 45 nucleotides.

In another particular embodiment, alone or in combination with other particular embodiments, the polynucleotide of the invention comprises, or consists of, sequence SEQ ID NO. 61.
SEQ ID NO. 61
ATGGACAAGCCCTCTAACCCCTCTTCCCATGAGTGA

The polynucleotide of the invention can be obtained by a skilled person by means of using techniques widely known in the state of the art. The sequence of the polynucleotide of the invention may be comprised in a gene construct.

Therefore, in another aspect, the invention relates to a gene construct, hereinafter the "gene construct of the invention", which comprises the polynucleotide of the invention.

Preferably, the construct comprises the polynucleotide of the invention operably linked to regulatory sequences for the expression of the polynucleotide of the invention. In theory, any promoter can be used in the gene constructs of the present invention, provided that said promoter is compatible with the cells in which the polynucleotide is to be expressed.

A promoter, or promoter region, is a nucleotide sequence that controls the transcription of a given gene (nucleotide sequences). The present invention relates to a nucleotide sequence that controls the transcription of the polynucleotide of the invention. Promoter sequences can be unidirectional or bidirectional. A unidirectional promoter is one that controls the transcription of one or more genes that are located in tandem with the first.

"In tandem" refers to the 3' end of the first gene that is followed, either consecutively or separated by a particular nucleotide sequence, by the 5' end of the second gene. A bidirectional promoter refers to the promoter region that controls transcription in two opposite directions, for example, the sequence preceding the kivD gene of *L. lactis* IFPL730 which acts as a bidirectional promoter. In other words, a bidirectional promoter directs the transcription of two divergently located genes, i.e., in the opposite direction, where the 5' end of both nucleotide sequences are closer to each other than the 3' end. In the present invention, the terms "promoter" and "promoter region" are used interchangeably. Furthermore, the promoters in the present invention may be constitutive or inducible. The term "inducible", as used in the present description, refers to the possibility that the promoter has a control element that allows it to activate or deactivate (repress) the transcription of the gene it regulates, in the presence of a factor external to the promoter.

Examples of promoters that can direct the transcription of the nucleotide of the invention include, but are not limited to, pT7, plac, ptrc, ptac, pBAD, or ret. Other suitable sequences which the gene construct may comprise are sequences which control and regulate said transcription and, if applicable, the translation of the product of interest such as, for example, transcription initiation and termination signals (tlt2, etc.), polyadenylation signal, origin of replication, ribosome binding sequences (RBS), transcription regulatory coding sequences (enhancers), transcriptional silencers (silencers), repressors, etc.

Additionally, the gene construct of the invention may contain markers or tags that allow the peptide of the invention to be isolated once it is synthesised in the cell.

Moreover, the polynucleotide or the gene construct of the invention may be part of a vector. Therefore, in another aspect, the invention relates to a vector, hereinafter "vector of the invention", which comprises the polynucleotide or the gene construct of the invention.

Examples of suitable expression vectors can be selected according to the conditions and needs of each specific case from expression plasmids, viral vectors (DNA or RNA), cosmids, artificial chromosomes, etc., which may further contain markers that can be used to select cells transfected or transformed with the gene or genes of interest. The choice of the vector will depend on the host cell and on the type of intended use. Therefore, according to a particular embodiment of the present invention, said vector is a plasmid or a viral vector. Said vector can be obtained by conventional methods known by those skilled in the art, just as different widely known methods such as, without limitation, chemical transformation, electroporation, microinjection, among others, can be used for microorganism and eukaryotic cell transformation.

In a more particular or preferred embodiment, the vector is a lentivirus-type viral vector.

The vector of the invention can be used to transform, transfect, or infect cells susceptible to being transformed, transfected, or infected by said vector. Said cells may be prokaryotic or eukaryotic cells. By way of example, the vector into which the nucleotide sequence is introduced, preferably DNA, can be a plasmid or a vector that, when introduced into a host cell, integrates into the genome of said cell and is replicated together with the chromosome (or chromosomes) into which it has integrated. Said vector can be obtained by conventional methods known to a person skilled in the art.

Therefore, another aspect of the invention relates to a cell, hereinafter the "cell of the invention", which comprises the polynucleotide, the gene construct, or the vector of the invention, for which said cell has been able to be transformed, transfected, or infected with a construct or a vector provided by this invention. Transformed, transfected, or infected cells can be obtained by conventional methods known by those skilled in the art. In a particular embodiment, said host cell is an animal cell transfected or infected with a suitable vector.

Host cells suitable for the expression of the peptide of the invention include, but are not limited to, mammalian, plant, insect, fungal, and bacterial cells. Bacterial cells include, but are not limited to, cells of Gram-positive bacteria such as species from the genera *Bacillus, Streptomyces,* and *Staphylococcus* and cells of Gram-negative bacteria such as cells of the genera *Escherichia* and *Pseudomonas.* Fungal cells preferably include yeast cells such as *Saccharomyces, Pichia pastoris,* and *Hansenula polymorpha.* Insect cells include, but are not limited to, Drosophila cells and Sf9 cells. Plant cells include, among others, cells of crops such as cereals, medicinal plants, ornamental plants, or bulb plants. Mammalian cells suitable for in the present invention include human keratinocyte cell lines, rat lung epithelial cells, lymphoblasts, human haematopoietic stem and progenitor cells.

In a more particular embodiment, alone or in combination with other particular embodiments, the cell of the invention is a mammalian cell, even more particularly wherein the mammalian cell is selected from the list consisting of human keratinocyte, rat lung epithelial cell, human lymphoblast, human haemotopoietic stem and progenitor cell. Preferably, the cell of the invention is a human keratinocyte or a rat lung epithelial cell.

### Composition of the invention

Likewise, the peptide, the polynucleotide, the gene construct, the vector, or the cell of the invention can be part of a composition, preferably a pharmaceutical composition, as an active agent or ingredient.

Therefore, another aspect of the invention relates to a composition, hereinafter the "composition of the invention", which comprises the peptide, the polynucleotide, the gene construct, the vector, or the cell of the invention. In a particular embodiment, the composition of the invention is a pharmaceutical composition.

In a particular embodiment, the composition of the invention comprises the peptide, the polynucleotide, the gene construct, the vector or the cell of the invention in a therapeutically effective amount, and a carrier that, in the case of a pharmaceutical composition, will be a pharmaceutically acceptable carrier.

In the present invention, "pharmaceutical composition" is understood to be any pharmaceutical preparation or form, which composition formula expressed in units of the international system, consists of a substance or mixture of substances, with constant weight, volume, and percentages, produced in legally established pharmaceutical laboratories, packaged, or labelled to be distributed and marketed as effective for diagnosis, treatment, mitigation, and prophylaxis of a disease, physical anomaly, or symptom, or the restoration, correction, or modification of the balance of organic functions in humans and animals. The pharmaceutical composition can be prepared by any of the methods described in the state of the art.

In the sense used in this description, the term "therapeutically effective amount" refers to the amount of the compound or of the pharmaceutical composition of the invention that will produce the desired effect and, in general, will be determined, among other causes, by the characteristics of said compound or said pharmaceutical composition and the therapeutic effect to be achieved. The dose for obtaining a therapeutically effective amount depends on a variety of factors such as, for example, age, weight, sex, or tolerance, of the mammal. The "adjuvants" and "pharmaceutically acceptable carriers" that can be used in said compositions are the carriers known in the state of the art.

The term "carrier" refers to a diluent or excipient with which the active ingredient is administered. Such carriers may be sterile liquids, such as water and oils, including those of petroleum, animal, plant, or synthetic origin, such as peanut oil, soya oil, mineral oil, sesame oil, and the like. Water or aqueous saline solutions and aqueous dextrose and glycerol solutions are preferably used as carriers, particularly for injectable solutions. Preferably, in the case of pharmaceutical carriers, they are approved by a state or federal government regulatory agency or are listed in the United States Pharmacopeia or other generally recognised pharmacopeia for use in animals, and more particularly in humans. The carriers and auxiliary substances necessary to manufacture the desired pharmaceutical form of administration of the pharmaceutical composition of the invention will depend, among other factors, on the chosen pharmaceutical form of administration. Said pharmaceutical forms of administration of the pharmaceutical composition will be manufactured according to conventional methods known to the person skilled in the art.

Furthermore, in the present invention, it is contemplated that the peptide of the invention may be associated with distribution systems or molecular carriers, including, but not limited to, exosomes and microvesicles. Given their properties, these distribution systems or molecular carriers are capable of crossing cell membranes and delivering their "load", the peptide of the invention in the present invention, in a biologically active form, and furthermore, they can cross biological membranes such as the blood-brain barrier. Therefore, in a preferred embodiment, the composition of the invention further comprises exosomes and/or microvesicles.

The compositions of the present invention may be formulated for administration thereof to an animal, and more preferably to a mammal, including a human, in a variety of ways known in the state of the art. Therefore, they may be, without limitation, in aqueous or non-aqueous solutions, in emulsions, or in suspensions. Examples of non-aqueous solutions are, for example, but not limited to, propylene glycol, polyethylene glycol, vegetable oils, such as olive oil, or injectable organic esters, such as ethyl oleate. Examples of aqueous solutions are, for example, but not limited to, water, alcoholic solutions in water, or saline media. Aqueous solutions may be buffered or not, and they may have additional active or inactive components. Additional components include salts to modulate ionic strength, preservatives including, but not limited to, antimicrobial agents, antioxidants, chelating agents, or the like, or nutrients, including glucose, dextrose, vitamins, and minerals. Alternatively, the compositions may be prepared for administration thereof in solid form. The compositions may be combined with various inert carriers or excipients, including, but not limited to: binders, such as microcrystalline cellulose, gum tragacanth, or gelatin; excipients, such as starch or lactose; dispersing agents, such as alginic acid or corn starch; lubricants, such as magnesium stearate, glidants such as colloidal silicon dioxide; sweetening agents, such as sucrose or saccharin; or flavouring agents, such as mint or methyl salicylate.

Additionally, the composition of the invention may comprise an adjuvant. "Adjuvant" is understood to be any substance that enhances the effectiveness of the pharmaceutical composition of the invention. Examples of adjuvants include, but are not limited to, adjuvants formed by aluminium salts (alum), such as aluminium hydroxide, aluminium phosphate or aluminium sulphate, oil-in-water or water-in-oil emulsion formulations such as Freund's Complete Adjuvant (FCA) as well as Freund's Incomplete Adjuvant (FIA), mineral gels; block copolymers, Avridine^{™}, SEAM62, adjuvants formed by components of the bacterial cell wall such as adjuvants including liposaccharides (e.g., lipid A or monophosphoryl lipid A (MLA), trehalose dimycolate (TDM), and components of the cell wall skeleton (CWS)), heat shock proteins or derivatives thereof, adjuvants derived from ADP-ribosylating bacterial toxins, including diphtheria toxin (DT), pertussis toxin (PT), cholera toxin (CT), *E. coli* heat-labile toxins (LT1 and LT2), *Pseudomonas* endotoxin A and exotoxin, B. *cereus* exoenzyme B, B. *sphaericus* toxin, *C. botulinum* toxins C2 and C3, *C. limosum* exoenzyme as well as the toxins of C. *perfringens, C. spiriforma* and C. *difficile,* S. *aureus,* EDIM, and mutants of mutant toxins such as CRM-197, non-toxic mutant of diphtheria toxin; saponins such as ISCOMs (immunostimulating complexes), chemokines, and cytokines such as interleukins (IL-1, IL-2, IL-4, IL-5, IL-6, IL-7, IL-8, IL-12, etc.), interferons (such as interferon gamma), macrophage colony-stimulating factor (M-CSF), tumour necrosis factor (TNF), defensins 102, RANTES, MIPI-alpha, and MEP-2, muramyl peptides such as N-acetyl-muramyl-L-threonyl-D-isoglutamine (thr-MDP), N-acetyl-nor-muramyl-L-alanyl-D-isoglutamine (nor-MDP), N-acetyl-muramyl-L-alanyl-D-isoglutaminyl-L-alanine-2-(1'-2'-dipalmitoyl-s-n-glycero-3 hydroxyphosphoryloxy)ethylamine (MTP-PE), etc.; adjuvants derived from the family of CpG molecules, CpG dinucleotides and synthetic oligonucleotides comprising CpG motifs, *C. limosum* exoenzyme and synthetic adjuvants such as PCPP, cholera toxin, Salmonella toxin, alum, and the like, aluminium hydroxide, N-acetyl-muramyl-L-threonyl-D-isoglutamine (thr-MDP), N-acetyl-nor-muramyl-L-alanyl-D-isoglutamine, MTP-PE and RIBI, containing three components extracted from bacteria, monophosphoryl lipid A, trehalose dimycolate and cell wall skeleton (MPL+TDM+CWS) in an emulsion of 12% squalene/Tween 80. Other examples of adjuvants include DDA (dimethyldioctadecylammonium bromide), Freund's Complete and Incomplete Adjuvants, QuilA, microvesicles, and exosomes.

The term "pharmaceutically acceptable" refers to the fact that the carrier or excipient must allow the activity of the compounds in the pharmaceutical composition, particularly of the peptide of the invention, that is, it is compatible with said components, so that it does not cause harm to the organisms to which it is administered.

Said compositions and/or their formulations may be administered to an animal, including a mammal and, therefore, a human, in a variety of ways, including, but not limited to, intraperitoneal, intravenous, intramuscular, subcutaneous, intrathecal, intraventricular, intraarticular, intratumoural, oral, enteral, parenteral, intranasal, intratracheal inhalation, ocular, or topical. In a particular embodiment, the composition of the invention is formulated for oral, parenteral, topical, nasal, intratracheal inhalation, intraarticular, sublingual, or intratumoral administration.

### Therapeutic uses of the invention

As explained herein above, the peptide of the invention, as well as the aspects associated therewith, the polynucleotide, the gene construct, the vector, the cell, or the composition of the invention, preferably the pharmaceutical composition of the invention, exhibit different activities with potential therapeutic use in subjects.

Therefore, another aspect of the invention relates to the peptide, the polynucleotide, the gene construct, the vector, the cell, or the composition of the invention for use as a medicament.

The term "medicament", as it is used herein, refers to any composition/substance used for the prevention, diagnosis, relief, treatment, or cure of diseases in a subject or which can be administered to the subject in order to restore, correct, or modify their physiological functions by exerting a pharmacological, immunological, or metabolic action.

In another aspect, the invention relates to the peptide, the polynucleotide, the gene construct, the vector, the cell, or the composition of the invention, for use in the prevention and/or treatment of, at least, one disease or disorder caused by cellular DNA damage in a subject.

In the present invention, "treatment" is understood to be the set of means used to treat, alleviate, or cure a disease.

The term "prevention" can be defined the set of means or measures aimed to prevent the onset of a disease, illness, or disorder, or to delay its progress.

In the present invention, "subject" is understood to be any animal, preferably a mammal, more preferably a primate, in particular, a human, of any race, sex, or age.

Examples of diseases or disorders caused by cellular DNA damage in a subject include, but are not limited to, dyskeratosis congenita, cri du chat syndrome, ataxia-telangiectasia, Nijmegen breakage syndrome, Bloom's syndrome, Werner's syndrome, Fanconi anaemia, ulcerative colitis, vascular ageing, arteriosclerosis, atherosclerosis, Duchene muscular dystrophy, progeria, hypersensitivity to light, genetic instability caused by mutation or an external agent, Hutchinson-Gilford syndrome, xeroderma pigmentosum, Rothmund-Thomson syndrome, pulmonary fibrosis, rheumatoid arthritis, disease(s) with chronic inflammation, or neurodegenerative diseases such as Huntington's disease, Alzheimer's disease, Parkinson's disease, cerebellar ataxia, and spinal cord degeneration, or diseases affecting the skin such as radiation dermatitis, chemotherapy-induced dermatitis, xeroderma pigmentosum, atopic dermatitis, systemic lupus erythematosus, dermomyositis, blistering diseases, psoriasis, contact dermatitis, systemic sclerosis, scleroderma, chronic urticaria, pemphigus, keloid scars, acne, and seborrhoeic dermatitis.

Therefore, in a particular embodiment, alone or in combination with other particular embodiments, the disease is selected from the list consisting of dyskeratosis congenita, cri du chat syndrome, ataxia-telangiectasia, Nijmegen breakage syndrome, Bloom's syndrome, Werner's syndrome, Fanconi anaemia, ulcerative colitis, vascular ageing, arteriosclerosis, atherosclerosis, Duchene muscular dystrophy, progeria, hypersensitivity to light, genetic instability caused by mutation or an external agent, Hutchinson-Gilford syndrome, xeroderma pigmentosum, Rothmund-Thomson syndrome, pulmonary fibrosis, rheumatoid arthritis, a disease with chronic inflammation, a neurodegenerative disease selected from the list consisting of Huntington's disease, Alzheimer's disease, Parkinson's disease, cerebellar ataxia, and spinal cord degeneration, and a disease affecting the skin selected from the list consisting of radiation dermatitis, chemotherapy-induced dermatitis, xeroderma pigmentosum, atopic dermatitis, systemic lupus erythematosus, dermomyositis, blistering diseases, psoriasis, contact dermatitis, systemic sclerosis, scleroderma, chronic urticaria, pemphigus, keloid scars, acne, and seborrhoeic dermatitis.

The peptide of the invention, as well as the polynucleotide, the gene construct, the vector, the cell, or the composition of the invention, preferably the pharmaceutical composition of the invention, can be used in the prevention and/or treatment of diseases or disorders related to DNA damage affecting the skin. Therefore, in a particular embodiment, alone or in combination with other particular embodiments, the disease or disorder caused by DNA damage in the subject affects the skin.

Examples of diseases or disorders caused by DNA damage in the subject affecting the skin include, but are not limited to, radiation dermatitis, chemotherapy-induced dermatitis, xeroderma pigmentosum, atopic dermatitis, systemic lupus erythematosus, dermomyositis, blistering diseases, psoriasis, contact dermatitis, systemic sclerosis, scleroderma, chronic urticaria, pemphigus, keloid scars, acne, or seborrhoeic dermatitis.

Therefore, in a more particular embodiment, the disease or disorder caused by DNA damage in the subject affecting the skin is selected from the list consisting of: radiation dermatitis, chemotherapy-induced dermatitis, xeroderma pigmentosum, atopic dermatitis, systemic lupus erythematosus, dermomyositis, blistering diseases, psoriasis, contact dermatitis, systemic sclerosis, scleroderma, chronic urticaria, pemphigus, keloid scars, acne, and seborrhoeic dermatitis.

Cellular DNA damage in the subject may be caused, in a non-limiting manner, basally or naturally, or by the action of an external agent. In a particular embodiment, alone or in combination with any one of the particular embodiments, DNA damage in the subject is caused by an external agent.

In addition to demonstrating the activity of the peptide of the invention on cellular DNA damage, the inventors have also demonstrated its effect on the expression of TERT and TERC genes, encoding components of the telomerase complex, specifically, an increase in the expression of both, therefore being applicable in the prevention and/or treatment of diseases or symptoms caused by telomere shortening in a subject.

Therefore, in another aspect, the invention relates to the peptide, the polynucleotide, the gene construct, the vector, the cell, or the composition of the invention, for use in the prevention and/or treatment of at least one disease or symptom caused by telomere shortening in a subject.

Examples of diseases or symptoms caused by telomere shortening include, but are not limited to, myelodysplasia, acute myeloid leukaemia, pulmonary fibrosis, nodular regenerative hyperplasia of the liver, cirrhosis, some neurodegenerative diseases such as secondary peripheral neuropathy, multiple neuropathy, or cellular ageing syndromes such as dyskeratosis congenita, idiopathic pulmonary fibrosis, and aplastic anaemia.

Therefore, in a particular embodiment, alone or in combination with other particular embodiments, the disease or symptom caused by telomere shortening is selected from the list consisting of myelodysplasia, acute myeloid leukaemia, pulmonary fibrosis, nodular regenerative hyperplasia of the liver, cirrhosis, a neurodegenerative disease selected from the list consisting of secondary peripheral neuropathy, multiple neuropathy, and a cellular ageing syndrome selected from the list consisting of dyskeratosis congenita, idiopathic pulmonary fibrosis, and aplastic anaemia.

As known in the state of the art, human cell senescence correlates with telomere erosion. Normal, untransformed cells are mortal due to telomere shortening in each of the cell division processes, such that telomeres can act as a biological clock, regulating cellular ageing. Therefore, the present invention is also applicable in the prevention and/or treatment of diseases or pathologies caused by an impaired senescence process. Therefore, in another aspect, the invention relates to the peptide, the polynucleotide, the gene construct, the vector, the cell, or the composition of the invention, for use in the prevention and/or treatment of a pathological disease or situation caused by an impaired senescence process, preferably wherein said process is mediated by telomere shortening.

As it is used in the present invention, the term "pathological disease or situation caused by an impaired senescence process", that is either normal or accelerated, refers to a disease in which cells and/or tissues exhibit a natural decrease, due to ageing, or accelerated decrease in proliferation capacity. Telomere shortening, or secondary structure loss thereof, causes cells to enter into senescence and/or apoptosis. These two processes act as biological checkpoints which prevent uncontrolled cell division and genetic instability. Defects in telomere length regulation are involved in the pathology of different diseases such as premature ageing syndromes. Therefore, this term, as it is used in the present invention, refers to those physiological situations altered by the natural ageing of human cells, preferably epithelial cells or highly proliferative cells belonging, by way of illustration and without limiting the scope of the invention, to the following group: skin cells, preferably cells from the epidermis, intestinal epithelium, cornea, liver, lung, hair bulb, etc., or cells from the hematopoietic system, preferably lymphocytes, macrophages, and erythrocytes; testicular and ovarian germ cells, etc., respectively, whether somatic cells, stem cells, or embryonic cells.

Examples of human diseases or pathological processes in which the control of senescence process and/or telomerase activity can be found, in a non-limiting illustrative manner, in reviews by Blasco MA, Telomere length, stem cells and ageing. Nature Chemical Biology 3 (10): 640-649, 2007; Crabbe L, Jauch A, Naeger CM, Holtgreve-Grez H., and Karlseder J. Telomere dysfunction as a cause of genomic instability in Werner syndrome. PNAS 104 (7): 2205-2210, 2007; Armanios, M. The Role of Telomeres in Human Disease. Annual Review of Genomics and Human Genetics, 23: 21-1-21.19. 2022; , Kam, M.L.W., Nguyen, T.T.T., Ngeow, Y.Y. Telomere biology disorders. Genomic Medicine, 6:36, 2021.

Another effect of the peptide of the invention refers to the inhibition of TGF-βresponse, particularly with decrease in the expression of the TGFβR1/2 receptor and MCP1 cytokine (monocyte chemoattractant protein-1, also referred to as "CCL2"). TGF-β response mediates processes such as fibrosis or epithelial/mesenchymal transition.

Thus, in another aspect, the invention relates to the peptide, the polynucleotide, the gene construct, the vector, the cell, or the composition of the invention, for use in the prevention and/or treatment of disorders or diseases comprising fibrosis in a subject, preferably wherein the fibrosis comprises elevated levels of TGF-β and/or cytokine MCP1.

In a particular embodiment, the disease comprising fibrosis is idiopathic pulmonary fibrosis.

Furthermore, the peptide of the invention has also been demonstrated to have an effect on decreasing the expression of interleukin 6 (IL-6), one of the main known pro-inflammatory molecules.

Therefore, another aspect of the invention relates to the peptide, the polynucleotide, the gene construct, the vector, the cell, or the composition of the invention, for use in the prevention and/or treatment of inflammatory disorders or diseases in a subject. Preferably, the inflammatory disorder or disease comprises elevated levels of IL-6.

Examples of diseases linked to elevated levels of IL-6 include, but are not limited to, rheumatoid arthritis, arthrosis, chronic inflammation, chronic obstructive pulmonary disease (COPD), pancreatitis, systemic lupus erythematosus, or systemic sclerosis. Therefore, in a particular embodiment, alone or in combination with other particular embodiments, the disorder or disease is selected from the list consisting of rheumatoid arthritis, arthrosis, chronic inflammation, chronic obstructive pulmonary disease (COPD), pancreatitis, systemic lupus erythematosus, and systemic sclerosis.

Terms used in these aspects of the invention, such as "prevention" and "treatment", "subject", as well as preferred embodiments thereof or of the mentioned diseases and disorders, have already been described previously, being likewise applicable to the present aspects of the invention.

### Uses of the peptide of the invention, and aspects derived from same, in the manufacture of a medicament.

In another aspect, the invention relates to the use of the peptide, polynucleotide, gene construct, vector, or cell of the invention in the preparation of a pharmaceutical composition or medicament. The techniques and methods for preparing pharmaceutical compositions have already been described herein above.

In another aspect, the invention relates to the use of the peptide, polynucleotide, gene construct, vector, or cell of the invention in the preparation of a pharmaceutical composition or medicament.

In another aspect, the invention relates to the use of the peptide, polynucleotide, gene construct, vector, or cell of the invention in the preparation of a pharmaceutical composition or medicament for the prevention and/or treatment of at least one disease or disorder caused by cellular DNA damage in a subject.

The terms "prevention" and "treatment", "subject", as well as examples of diseases or disorders caused by cellular DNA damage, have already been explained above, and these terms and their preferred embodiments are applicable to the present aspect the invention.

In another aspect, the invention relates to the use of the peptide, polynucleotide, gene construct, vector, or cell of the invention in the preparation of a pharmaceutical composition or medicament for the prevention and/or treatment of at least one disease or symptom caused by telomere shortening in a subject.

The terms "prevention" and "treatment", "subject", as well as examples of diseases or symptoms caused by telomere shortening, have already been explained above, and these terms and their preferred embodiments are applicable to the present aspect the invention.

In another aspect, the invention relates to the use of the peptide, polynucleotide, gene construct, vector, or cell of the invention in the preparation of a pharmaceutical composition or medicament for the prevention and/or treatment of a pathological disease or situation caused by an impaired senescence process, preferably wherein said process is mediated by telomere shortening.

In another aspect, the invention relates to the use of the peptide, polynucleotide, gene construct, vector, or cell of the invention in the preparation of a pharmaceutical composition or medicament for the prevention and/or treatment of disorders or diseases comprising fibrosis in a subject, preferably wherein the fibrosis comprises elevated levels of TGF-β and/or cytokine MCP1.

In another aspect, the invention relates to the use of the peptide, polynucleotide, gene construct, vector, or cell of the invention in the preparation of a pharmaceutical composition or medicament for the prevention and/or treatment of inflammatory disorders or diseases in a subject. Preferably, the inflammatory disorder or disease comprises elevated levels of IL-6.

Terms used in these aspects of the invention, such as "prevention" and "treatment", "subject", as well as preferred embodiments thereof or of the mentioned diseases and disorders, have already been described previously, being likewise applicable to the present aspects of the invention.

### In vitro uses of the peptide of the invention

In addition to the therapeutic applications mentioned above, the application of the peptide of the invention, as well as the inventive aspects derived therefrom, in *in vitro* assays, is also possible. Therefore, another aspect of the invention relates to the use of the peptide, polynucleotide, gene construct, vector, cell, or composition of the invention for the *in vitro* inhibition of epithelial/mesenchymal transition in cells.

In the present invention, "epithelial/mesenchymal transition" is understood to be a process whereby an epithelial cell temporarily acquires the phenotype of a mesenchymal cell as response to an internal or external stimulus (Hay ED. An overview of epithelio-mesenchymal transformation. Acta Anat (Basel). 1995;154:8-20.). This process may be mediated by TGF-β secretion.

In a particular embodiment, the epithelial/mesenchymal transition is mediated by TGF-β or comprises elevated levels of TGF-β and/or the cytokine MCP1.

On the other hand, as a result of the activities and effects associated with the peptide of the invention, such as the protection or reduction effect against genetic damage, the peptide and its associated aspects can be used *in vitro* to improve cell viability.

Therefore, another aspect of the invention relates to the *in vitro* use of the peptide, polynucleotide, gene construct, vector, cell, or composition of the invention to improve cell viability.

In a more particular embodiment, alone or in combination with other particular embodiments of any of the *in vitro* uses, the cell is a mammalian cell, even more particularly human keratinocyte or rat lung epithelial cell.

### Kit of the Invention

The administration of the peptide, polynucleotide, gene construct, vector, cell, or pharmaceutical composition of the invention may require a number of components, which in turn can be arranged together in the form of a kit.

Therefore, in another aspect, the invention relates to a kit, hereinafter the "kit of the invention", which comprises the peptide, polynucleotide, gene construct, vector, cell, or composition of the invention.

Useful components for the administration thereof and that may be included in the kit include, but are not limited to, buffer solution, lysis solution, sterile material (such as syringes, swabs, cotton balls, or clamps), distilled water or alcohols (ethanol). Additionally, the kit may contain instructions or directions to guide the person skilled in the art in the administration thereof. These instructions may be present in the mentioned kits in a variety of forms, one or more of which may be present in the kit. One form in which these instructions may be present is as printed information on a suitable medium or substrate, e.g., a sheet or sheets of paper on which the information is printed, in the kit packaging, in a package insert, etc. Another medium would include a computer-readable medium, for example, a CD, a USB, etc., in which the information has been recorded. Another medium that may be present is a website address that can be used through the Internet to access the information at a remote site. Any suitable medium can be present in the kits.

The kit of the invention, which can be used in the administration of the peptide, polynucleotide, gene construct, vector, cell, or composition of the invention, can also be used in *in vitro* assays.

Therefore, another aspect of the invention relates to the use of the kit of the invention for the *in vitro* inhibition of epithelial/mesenchymal transition in cells.

Another aspect of the invention relates to the *in vitro* use of the kit of the invention for improving cell viability.

The terms used to define the kit and uses of the kit of the invention have already been explained, and these terms and their preferred embodiments are applicable to the different uses of the kit of the invention.

### Treatment/prevention method of the invention

In another aspect, the invention relates to a method for the treatment and/or the prevention of at least one disease or disorder caused by cellular DNA damage in a subject, which comprises administering to said subject the peptide, polynucleotide, gene construct, vector, cell, or composition of the invention.

Another aspect of the invention relates to a method for the prevention and/or treatment of at least one disease or symptom caused by telomere shortening in a subject, which comprises administering to said subject the peptide, polynucleotide, gene construct, vector, cell, or composition of the invention.

Another aspect of the invention relates to a method for the prevention and/or treatment of a pathological disease or situation caused by an impaired senescence process, preferably wherein said process is mediated by telomere shortening, which comprises administering to said subject the peptide, polynucleotide, gene construct, vector, cell, or composition of the invention.

Another aspect of the invention relates to a method for the prevention and/or treatment of disorders or diseases comprising fibrosis in a subject, which comprises administering to said subject the peptide, polynucleotide, gene construct, vector, cell, or composition of the invention.

Another aspect of the invention relates to a method for the prevention and/or treatment of inflammatory disorders or diseases in a subject, which comprises administering to said subject the peptide, polynucleotide, gene construct, vector, cell, or composition of the invention.

The terms defined and explained for the remaining aspects of the invention, as well as the preferred embodiments thereof are also applicable to the treatment and/or prevention method of the invention.

### DESCRIPTION OF THE DRAWINGS

**Figure 1****.** Effect of the expression of the peptides on the response to basal DNA damage or DNA damage induced by bleomycin in RL6TN cells.
**Figure 2****.** Effect of the expression of the peptides on response to basal DNA damage or DNA damage induced by bleomycin in HaCaT cells.
**Figure 3****.** Effects of peptide transduction on the expression of the components of the TERT and TERC telomerase complex.
**Figure 4****.** Effect of peptide expression on epithelial/mesenchymal cellular transition.
**Figure** 5. Effect of the peptides on the expression of the proinflammatory cytokine interleukin 6 (IL-6).

### EXAMPLES

Next, the invention will be illustrated by means of assays carried out by the inventors which demonstrate the effectiveness of the invention.

The activity of a number of dyskerin protein-derived peptides and peptides partially related to the GSE24-2 and GSE4 peptides disclosed previously was analysed in the examples. The sequence of the analysed peptides is shown in Table 2.

**Table 2. Amino acid sequence of the peptides analysed in this patent.**

| Peptide | Amino acid sequence |
|---|---|
| P1 | NLDKPSNP (SEQ ID NO. 12) |
| P2 | NLDKPSNPSSHE (SEQ ID NO. 13) |
| P3 | DKPSNPSSHE (SEQ ID NO. 1) |
| P11 | GFINLDK (SEQ ID NO. 14) |
| P12 | DKPSNP (SEQ ID NO. 15) |
| P13 | EHSSPNSPKD (SEQ ID NO. 16) |

Materials used in these assays, such as, primers or other sequences, are shown in the following tables:

**Table 3. Oligonucleotides (primers) used in quantitative PCR (qPCR, quantitative polymerase chain reaction). "Forward" or "Reverse" refer to whether the primer is a forward or reverse primer, respectively.**

| Oligonucleotides/primers used for qPCR | Sequence (5' - 3') | SEQ ID NO. |
|---|---|---|
| Human TERT, forward | CGGAAGAGTGTCTGGAGCAA | 21 |
| Human TERT, reverse | GGATGAAGCGGAGTCTGG | 22 |
| Rat TERT, forward | GAGTGTCACTAAGGGGGCCAAG | 23 |
| Rat TERT, reverse | CAGCTGCAGTCTCTTAAGGAGG | 24 |
| Human TERC, forward | TCTAACCCTAACTGAGAAGGGCGTAG | 25 |
| Human TERC, reverse | GTTTGCTCTAGAATGAACGGTGGAAG | 26 |
| Rat TERC, forward | CGTGAAGAGCTAGTCTCTG | 27 |
| Rat TERC, reverse | GGTTGTAGGAACTGAGTTCC | 28 |
| Human TGFbR2, forward | CCAGCTTCTGGCTCAACCAACCAC | 29 |
| Human TGFbR2, reverse | CCAGAAGAGAGCTATTTGGTAGTG | 30 |
| Rat TGFbR1, forward | AGAAAGCATCGGCAAAGGTC | 31 |
| Rat TGFbR1, reverse | CCCAGGATATTTTTTCATGGCG | 32 |
| Human MCP1, forward | CAAGGGCTCGCTCAGCCAG | 33 |
| Human MCP1, reverse | CCTTGGCCCCACAATGGTCTTG | 34 |
| Rat MCP1, forward | GGCAAGATGATCCCAATGAG | 35 |
| Rat MCP1, reverse | CTGATCTCACTTGGTTTTCTGG | 36 |
| Human IL-6, forward | GCCAGAGAGCTGTGCAGATGAG | 37 |
| Human IL-6, reverse | CAGTGGACAGGTTTCTGACC | 38 |
| Rat IL-6, forward | CCTGGAGTTTGTGAAGAAGAACAAC | 39 |
| Rat IL-6, reverse | GGTCCTCCTTAGCCACTCCTTC | 40 |
| Human GAPDH, forward | GAGAGAGACCCTCACTCACTGCTG | 41 |
| Human GAPDH, reverse | GATGGGGTACATGACAAGGTGC | 42 |
| Rat GAPDH, forward | GCCAAGTATGATGACATCAAG | 43 |
| Rat GAPDH, reverse | GCTGTAGCCATATTCATTCATTGTC | 44 |

**Table 4. Polynucleotides used to generate lentiviral expression vectors of the peptides.**

| Polynucleotide according to peptide | Sequence (5' - 3') | SEQ ID NO. |
|---|---|---|
| Polynucleotide of peptide P1 | TCGACACATGAATGAATCTTGACAAGCCCTCTAA CCCCTGAT | 45 |
| | CTAGATCAGGGGTTAGAGGGCTTGTCAAGATTC ATG | 46 |
| Polynucleotide of peptide P2 | TCGACATGAATGAATCTTGACAAGCCCTCTAACC CCTCTTCCCATGAGTGAT | 47 |
| | CTAGATCACTCATCATGGGAAGAGGGGTTAGAG GGCTTGTCAAGATTCATG | 48 |
| Polynucleotide of peptide P3 | TCGACATGGACAAGCCCTCTAACCCCTCTCTTCC CATGAGTGAT | 49 |
| | CTAGATCACTCATCATGGGAAGAGGGGTTAGAG GGCTTGTCCATG | 50 |
| Polynucleotide of peptide P4 | TCGACATGCCCAACTCTCTCCCAAGGACCTTAAT ATTTTCGGTTGAT | 51 |
| | CTAGATCAACCGAAAATATTAAGGTCCTTGGGAG AGTTGGGCATG | 52 |
| Polynucleotide of peptide P11 | TCGACATGGGTTTCATTAATCTTGACAAGTGAT | 53 |
| | CTAGATCACTTGTCAAGATTAATGAAACCCATG | 54 |
| Polynucleotide of peptide P12 | TCGACATGGACAAGCCCTCTAACCCCTGAT | 55 |
| | CTAGATCAGGGGTTAGAGGGCTTGTCCATG | 56 |
| Polynucleotide of peptide P13 | TCGACATGGAGCATTCATTCCTCTCTCCCCCAAC TCTCCCAAGGACTGAT | 57 |
| | CTAGATCAGTCCTTGGGAGAGTTGGGAGGAATG CTCCATG | 58 |
| Polynucleotide of peptide GSE4 | TCGACCATGGGTTTCATTAATCTTGACAAGCCCT CTAACCCCTAAG | 59 |
| | CTAGACTTAGGGGTTAGAGGGCTTGTCAAGATTA ATGAAACCCATG | 60 |

The biological activities on which the effect of the peptides has been studied are described in the sections shown below.

### Example 1. Activity of the peptides on genetic damage induced by treatment of cultured cells with bleomycin.

Many environmental components affect the cells in our body body, causing DNA alterations with genotoxic effects. The most frequent condition is DNA strand breakage that triggers a complex cell repair response. One of the quickest responses consists of the incorporation of the phosphorylated H2AX histone variant, referred to as yH2AX, to the chromatin in the damaged DNA region.

In the experiment shown in Figure 1, rat lung epithelial cells RL6TN were transduced with lentiviral vectors directing the expression of the peptides under study and the corresponding controls. The presence of γH2AX was then determined by means of Western blot assays.

RL6TN cells were transduced with lentiviral vectors directing the expression of the different peptides under study (sequences derived from pCCL-hPGK.GSE4.wPREchim, SEQ ID NO. 62, in which the oligonucleotides corresponding to each peptide, shown in Table 4, SEQ ID Nos: 46-60, have been included between restriction sites Sall and Xbal) or of the GFP protein (green fluorescent protein) (PGK-EGFP sequence, SEQ ID NO. 63) as negative control. 600,000 non-transduced (control, CT) cells or cells transduced with the different lentiviral vectors were cultured for 6 hours in the absence (Panel A) or presence of 4 µg/mL of bleomycin (Merck, Burlington, MA. USA) (Panel B). After this culture period, the cells were lysed and the amount of yH2AX was determined by means of Western blot using an yH2AX-specific antibody (Cell Signalling Technology, Danvers, MA, USA). Expression of the β-actin protein as loading control was simultaneously determined using a specific antibody (Santa Cruz Biotechnology, Dallas, TX, USA). Protein expression was quantified using a luminometer. The expression of yH2AX in each sample was calculated based on the amount of β-actin. These relative values were normalised to the value corresponding to control cells not treated with bleomycin.

Figure 1A shows baseline expression in RL6TN cells transduced in culture. Figure 1B shows expression in transduced cells incubated with the antibiotic bleomycin which induces cellular DNA damage. The results obtained show that the expression of the P3 peptide causes a decrease in the levels of yH2AX, which are indicators of the existence of DNA damage, both in culture control conditions and after cell treatment with bleomycin (p<0.05 with respect to CT, *, Figure 1A, calculated by means of a one-way ANOVA test and Dunnett's post-hoc test). This decrease is greater than that caused by the expression of the GSE4 peptide described previously.

Other assayed peptides that are shorter than GSE4 do not cause decrease in the expression of yH2AX, so it is not obvious that peptides shorter than GSE4 maintain the same biological activity. In fact, these results indicated that the P3 peptide had the highest biological activity among the assayed peptides and the rest of the functional studies, which will be described below, focused on this peptide.

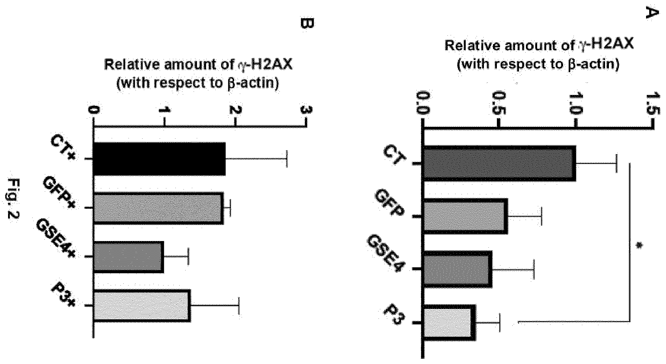

DNA damage signalling studies were reproduced in human skill keratinocyte cell line HaCaT. This cell line is often used to study skin's response to environmental and pathogenic agents. HaCaT cells were transduced with lentiviral vectors which allow the expression of the P3 peptide, the GSE4 peptide, as a positive control, or GFP as a negative control. 100,000 non-transduced (CT) cells or cells transduced with the various peptides were cultured in the absence (Figure 2A) or presence of 50 µg/mL of bleomycin (Figure 2B) for 6 hours. Subsequently, the cells were lysed and the amount of yH2AX present in the extracts was determined by Western Blot in the same way as indicated for the assays in RL6TN cells described above.

In this cell line, it is also observed that the P3 peptide reduces DNA damage signalling (p<0.05 with respect to CT, * Figure 2A, Calculated by means of a Student's test). In the case of baseline damage, the reduction is greater than that caused by the expression of GSE4.

### Example 2. Activity of the peptides on telomere shortening-mediated cellular ageing processes.

Some premature ageing syndromes, such as dyskeratosis congenita, idiopathic pulmonary fibrosis, or some cases of aplastic anaemia, are caused by accelerated telomere shortening due to mutations in the genes encoding proteins involved in the extension or protection thereof. They include genes encoding TERT and TERC components of the telomerase complex which is responsible for telomere extension. The expression of these genes is also reduced by environmental agents which induce cell ageing, such as exposure to ionising radiation. For this reason, the inventors studied the expression levels of these two genes in cells transduced with the expression vectors of the P3 peptide and the corresponding control peptides.

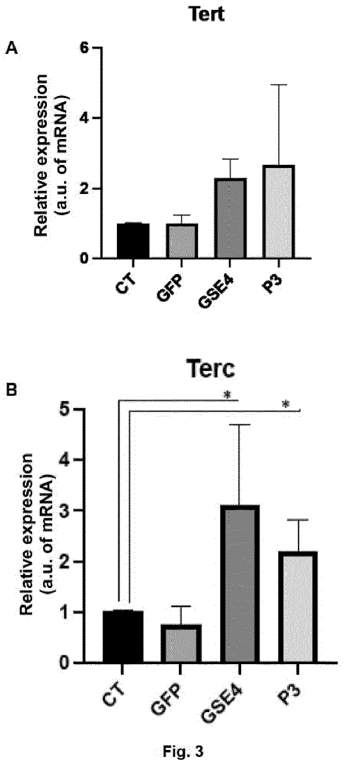

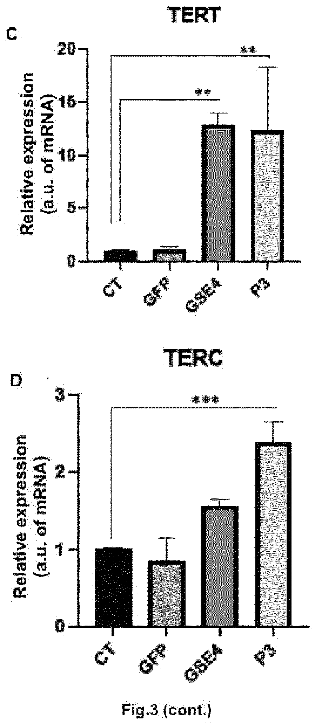

RNA was isolated from control cells or cells transduced with vectors expressing GFP or the GSE4 and P3 peptides. The corresponding cDNAs, which were used to determine the expression levels of the mRNA encoding TERT (Figure 3, panels A and C) and TERC (Figure 3, panels B and D) by means of quantitative PCR, were synthesised from these RNAs. Expression levels were corrected by levels corresponding to the mRNA encoding GAPDH, used as a control. Relative values were normalised with respect to values obtained for non-transduced control cells (CT). Panels A and B show the results obtained for RL6TN cells and panels C and D show the results corresponding to HaCaT cells.

For assays performed in RLE6TN cells, shown in Figures 3A and 3B, oligonucleotides corresponding to SEQ ID Nos: 23, 24 for TERT, SEQ ID Nos:27, 28 for TERC, and SEQ ID Nos:43, 44 for GAPDH were used in quantitative PCR assays.

In this RLE6TN line, a significant increase was observed in the expression of Terc for GSE4 (*, p<0.05, Figure 3B) and P3 (*, p<0.05, Figure 3B), calculated according to the Student's test. In the case of Tert, an increase was also observed in the expression for GSE4 and P3.

The results obtained for HaCaT cells are depicted in Figures 3C and 3D where oligonucleotides corresponding to SEQ ID Nos: 21, 22 for TERT, SEQ ID Nos: 25, 26 for TERC, and SEQ ID Nos: 41, 42 for GAPDH were used. The GSE4 and P3 peptides caused an increase in the expression of TERT and TERC. This difference is statistically significant for both peptides in the case of TERT (**, p<0.01, ANOVA test and Dunnett's post-hoc test) and for the P3 peptide in the case of TERC (***, p<0.001, ANOVA test and Dunnett's post-hoc test).

### Example 3. Effect of the peptides on fibrosis and epithelial/mesenchymal transition processes.

The ageing of some tissues is accompanied by fibrotic processes in which fibroblasts and myofibroblasts accumulate and in which epithelial/mesenchymal transition of epithelial cells is induced. This process occurs, for example, in idiopathic pulmonary fibrosis and in fibrosis processes as a result of skin exposure to ionising radiation. Many of these processes are mediated by the secretion of the cytokine TGFβ (transforming growth factorβ). The possible effect of the peptides on these processes was studied by incubating cells with TGFβ (R&D Systems, Bio-Techne, Minneapolis; MN, USA) and studying the expression of two mRNAs induced by this cytokine, i.e., the TGFβ receptor (TGFβR1/2) and the cytokine MCP1 (also referred to as CCL2) by means of reverse transcription of RNA and quantitative PCR.

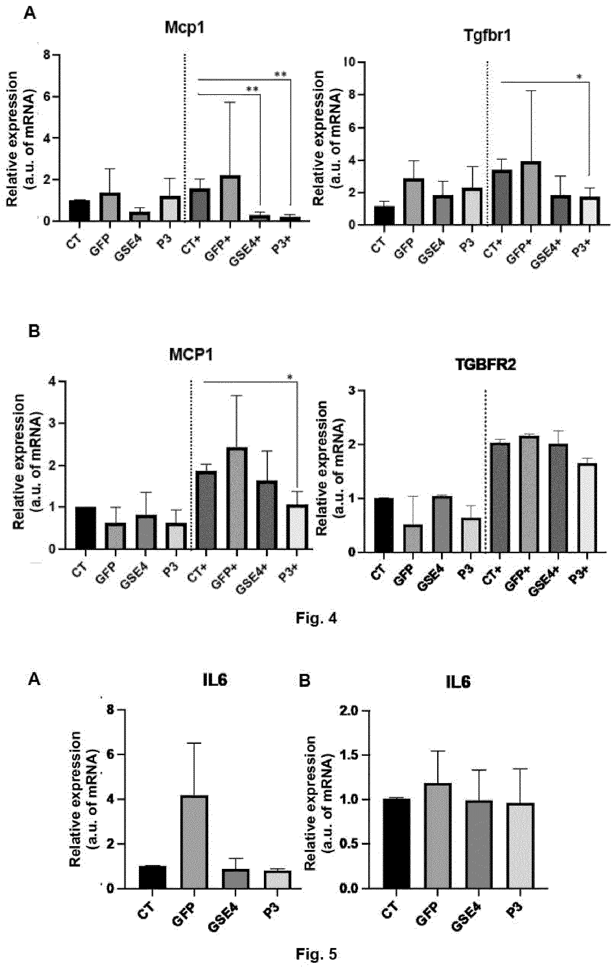

Control cells or cells transduced with GFP expression vectors, or the GSE4 and P3 peptides were cultured in the absence or presence of a concentration of 5 ng/mL of the TGFβ factor (identified with the symbol + in Figure 4) to induce epithelial/mesenchymal transition. After 48 of culture, RNA was obtained from the cells and converted into cDNA by means of reverse transcription. The expression levels of the mRNA encoding TGFBR-1/2 (TGFβ-receptor 1 or 2) and MCP1 (monocyte chemoattractant protein-1, also referred to as CCL2: C-C motif chemokine ligand 2) were determined by quantitative PCR. The values obtained were corrected by those corresponding to the control mRNA (GAPDH) and normalised by the expression in control cells (CT). Panel A of Figure 4 shows the results obtained for RL6TN cells (400,000 cells per sample) and panel B shows the results corresponding to HaCaT cells (500,000 cells per sample).

In the assays shown in Figures 4A, for RLE6TN cells, oligonucleotides SEQ ID Nos 31 and 32 for TGFBR1, 35 and 36 for MCP1, and 43 and 44 for GAPDH were used in quantitative PCR. To obtain the results shown in Figure 4B, for HaCaT cells, oligonucleotides SEQ ID Nos: 29 and 30 for TGFBR2, SEQ ID Nos: 33 and 34 for MCP1, and SEQ ID Nos: 41y 42 for GAPDH b were used.

Both graphs show how the expression of P3 peptide decreases the induction of expression of both markers after incubating the cells with TGFβ. The previously characterised GSE4 peptide also causes this inhibitory effect of the response to TGFβ. The differences observed in RLE6TN cells were statistically significant for the P3 peptide (** p<0.01 for Mcp1 and * p<0.05 for Tgfbr1, according to the Student's test) and for GSE4 in the case of Mcp1 (** p<0.01). The differences observed in HaCaT cells were significant for the P3 peptide and the expression of MCP1 (* p<0.05).

### Example 4. Modulation of pro-inflammatory response by the peptides.

Another important component of tissue ageing is the production of pro-inflammatory interleukins that induce the presence of basal inflammation in tissues. This inflammatory situation also occurs in some diseases of premature ageing and in response to environmental aggressions. Interleukin-6 (IL-6) is one of the main pro-inflammatory molecules and, for this reason, the possible effect of the expression of the peptides on the synthesis of this molecule was determined. In these experiments, the expression of the mRNA encoding IL-6 in cells transduced with the expression vectors of the P3 peptide and controls was determined.

RNA was obtained from RL6TN cells (Figure 5, panel A) or HaCaT cells (Figure 5, panel B) not transduced (CT) or transduced with lentiviral vectors expressing GFP or the GSE4 or P3 peptides. cDNA was synthesised from each RNA by reverse transcription and the amount of mRNA encoding IL-6 was quantified by quantitative PCR as described in Examples 2 and 3 above.

The obtained results are shown in Figures 5A (RLE6TN cells) and 5B (HaCaT cells). In the assays with RLE6TN cells, oligonucleotides with SEQ ID Nos: 39 and 40 for IL-6 and SEQ ID Nos: 43 and 44 for GAPDH were used for quantitative PCRs. In the assay performed with HaCaT cells, oligonucleotides with SEQ ID Nos: 37 and 38 for IL-6 and SEQ ID Nos: 41 and 42 for GAPDH were used.

It was observed in both cell types that, like the GSE4 peptide, the expression of the P3 peptide decreased the expression of IL-6 mRNA.

## Claims

1. A peptide comprising the amino acid sequence with SEQ ID NO. 1 with the proviso that said peptide does not have the amino acid sequence SEQ ID NO. 3, SEQ **ID** NO. 4, SEQ ID NO. 5, SEQ ID NO. 6, SEQ **ID** NO. 7, SEQ **ID** NO. 8, SEQ ID NO. 9, SEQ **ID** NO. 10, SEQ **ID** NO. 11 or SEQ ID NO. 64.

2. Peptide according to claim 1, wherein the peptide has a length of 10 to 14 amino acids.

3. Peptide according to claim 1 or 2, wherein the peptide comprises, at least, one nuclear localisation sequence attached to, at least, one of the carboxyl- or amino-terminal ends of its amino acid sequence.

4. Peptide according to claim 3, wherein the nuclear localisation sequence comprises the sequence KRKR (SEQ ID NO. 19) or the sequence KKEKKKSK (SEQ **ID** NO. 20)

5. A polynucleotide encoding a peptide according to any one of claims 1 to 4.

6. A gene construct comprising a polynucleotide according to claim 5.

7. A vector comprising a polynucleotide according to claim 5, or a gene construct according to claim 6.

8. Vector according to claim 7, wherein the vector is a viral vector, preferably a lentivirus-type viral vector.

9. A cell comprising a peptide according to any one of claims 1 to 4, a polynucleotide according to claim 5, a gene construct according to claim 6, or a vector according to claim 7 or 8.

10. A composition comprising a peptide according to any one of claims 1 to 4, a polynucleotide according to claim 5, a gene construct according to claim 6, a vector according to claim 7 or 8, or a cell according to claim 9.

11. Composition according to claim 10, further comprising a carrier.

12. Composition according to claim 10 or 11, wherein the composition is formulated for oral, parenteral, topical, nasal, intratracheal inhalation, intraarticular, or sublingual administration.

13. Composition according to any one of claims 10 to 12, wherein the composition is a pharmaceutical composition.

14. Peptide according to any one of claims 1 to 4, polynucleotide according to claim 5, a gene construct according to claim 6, vector according to claim 7 or 8, a cell according to claim 9, or composition according to any one of claims 10 to 13, for use thereof as a medicament.

15. Peptide according to any one of claims 1 to 4, polynucleotide according to claim 5, gene construct according to claim 6, vector according to claim 7 or 8, cell according to claim 9, or composition according to any one of claims 10 to 13, for use in the prevention and/or treatment of, at least, one disease caused by cellular DNA damage in a subject.

16. Peptide, polynucleotide, gene construct, vector, cell, or composition for use according to claim 15, wherein the disease is selected from the list consisting of dyskeratosis congenita, cri du chat syndrome, ataxia-telangiectasia, Nijmegen breakage syndrome, Bloom's syndrome, Werner's syndrome, Fanconi anaemia, ulcerative colitis, vascular ageing, arteriosclerosis, atherosclerosis, Duchene muscular dystrophy, progeria, hypersensitivity to light, genetic instability caused by mutation or an external agent, Hutchinson-Gilford syndrome, xeroderma pigmentosum, Rothmund-Thomson syndrome, pulmonary fibrosis, rheumatoid arthritis, a disease with chronic inflammation, a neurodegenerative disease selected from the list consisting of Huntington's disease, Alzheimer's disease, Parkinson's disease, cerebellar ataxia, and spinal cord degeneration, and a disease affecting the skin selected from the list consisting of radiation dermatitis, chemotherapy-induced dermatitis, xeroderma pigmentosum, atopic dermatitis, systemic lupus erythematosus, dermomyositis, blistering diseases, psoriasis, contact dermatitis, systemic sclerosis, scleroderma, chronic urticaria, pemphigus, keloid scars, acne, and seborrhoeic dermatitis.

17. Peptide according to any one of claims 1 to 4, polynucleotide according to claim 5, gene construct according to claim 6, vector according to claim 7 or 8, cell according to claim 9, or composition according to any one of claims 10 to 13, for use in the prevention and/or treatment of, at least, one disease or symptom caused by telomere shortening in a subject.

18. Peptide, polynucleotide, gene construct, vector, cell, or composition for use according to claim 17, wherein the disease or symptom caused by telomere shortening is selected from the list consisting of myelodysplasia, acute myeloid leukaemia, pulmonary fibrosis, nodular regenerative hyperplasia of the liver, cirrhosis, a neurodegenerative disease selected from the list consisting of secondary peripheral neuropathy, multiple neuropathy, and a cellular ageing syndrome selected from the list consisting of dyskeratosis congenita, idiopathic pulmonary fibrosis, and aplastic anaemia.

19. Peptide according to any one of claims 1 to 4, polynucleotide according to claim 5, gene construct according to claim 6, vector according to claim 7 or 8, cell according to claim 9, or composition according to any one of claims 10 to 13, for use in the prevention and/or treatment of disorders or diseases comprising fibrosis in a subject.

20. Peptide, polynucleotide, gene construct, vector, cell, or composition for use according to claim 19, wherein the fibrosis comprises elevated levels of TGF-β and/or cytokine MCP1.

21. Peptide according to any one of claims 1 to 4, polynucleotide according to claim 5, gene construct according to claim 6, vector according to claim 7 or 8, cell according to claim 9, or composition according to any one of claims 10 to 13, for use in the prevention and/or treatment of inflammatory disorders or diseases in a subject.

22. Peptide, polynucleotide, gene construct, vector, cell, or composition for use according to claim 21, wherein the inflammatory disorder or disease comprises elevated levels of IL-6.

23. Peptide according to any one of claims 1 to 4, polynucleotide according to claim 5, gene construct according to claim 6, vector according to claim 7 or 8, cell according to claim 9, or composition according to any one of claims 10 to 13, for use in the prevention and/or treatment of a disease or pathological situation caused by an impaired senescence process.

24. Peptide, polynucleotide, gene construct, vector, cell, or composition for use according to claim 23, wherein said process is mediated by telomere shortening.

25. Use of a peptide according to any one of claims 1 to 4, a polynucleotide according to claim 5, a gene construct according to claim 6, a vector according to claim 7 or 8, a cell according to claim 9, or a composition according to any one of claims 10 to 13, for the *in vitro* inhibition of epithelial/mesenchymal transition in cells.

26. *In vitro* use of a peptide according to any one of claims 1 to 4, a polynucleotide according to claim 5, a gene construct according to claim 6, a vector according to claim 7 or 8, a cell according to claim 9, or a composition according to any one of claims 10 to 13, for improving cell viability.

27. A kit comprising a peptide according to any one of claims 1 to 4, a polynucleotide according to claim 5, a gene construct according to claim 6, a vector according to claim 7 or 8, a cell according to claim 9, or a composition according to any one of claims 10 to 13.

28. Use of the kit according to claim 27, for the *in vitro* inhibition of epithelial/mesenchymal transition in cells.

29. *In vitro* use of the kit according to claim 27, for improving cell viability.
